(19) Europäisches Patentamt
European Patent Office
Office européen des brevets

(11) Publication number: **0 445 467 A1**

(12) **EUROPEAN PATENT APPLICATION**

(21) Application number: **90313104.3**

(22) Date of filing: **03.12.90**

(51) Int. Cl.⁵: **C07K 5/02**, C07K 5/06, A61K 37/64

The title of the invention has been amended (Guidelines for Examination in the EPO, A-III, 7.3).

(30) Priority: **08.12.89 GB 8927872**

(43) Date of publication of application: **11.09.91 Bulletin 91/37**

(84) Designated Contracting States: **AT BE CH DE DK ES FR GB GR IT LI LU NL SE**

(71) Applicant: **Beecham Group p.l.c.**
**SB House Great West Road**
**Brentford Middlesex TW8 9BD(GB)**

(72) Inventor: **Cassidy, Frederick, SmithKline Beecham Pharm.**
**Coldharbour Road, The Pinnacles**
**Harlow, Essex CM19 5AD(GB)**
Inventor: **Smith, Stephen Allan, SmithKline Beecham Pharm.**
**Coldharbour Road, The Pinnacles**
**Harlow, Essex CM19 5AD(GB)**
Inventor: **Ham, Peter, SmithKline Beecham Pharm.**
**Coldharbour Road, The Pinnacles**
**Harlow, Essex CM19 5AD(GB)**
Inventor: **Nash, David John, SmithKline Beecham Pharm.**
**Coldharbour Road, The Pinnacles**
**Harlow, Essex CM19 5AD(GB)**

(74) Representative: **Jones, Pauline et al**
**SmithKline Beecham, Corporate Patents,**
**Great Burgh, Yew Tree Bottom Road**
**Epsom, Surrey KT18 5XQ(GB)**

(54) **Renin inhibitors and antiviral agents.**

(57) Compounds of formula (I), and pharmaceutically acceptable salts thereof:

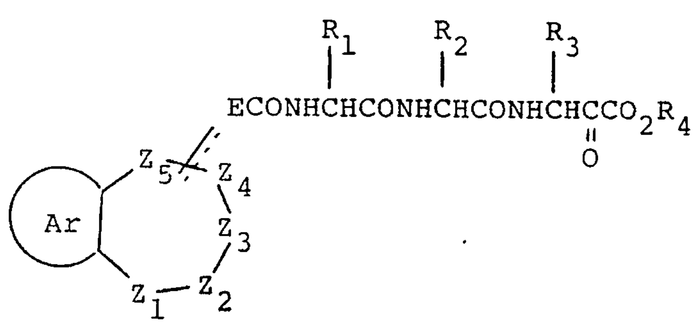

(I)

having renin inhibitor activity, a process for their preparation and their use as pharmaceuticals.

EP 0 445 467 A1

## PHARMACEUTICALS

The invention relates to novel compounds having pharmacological activity, to processes for their preparation, to pharmaceutical preparations containing them and to their use in medicine.

Renin is a natural enzyme, disorders in relation to which are implicated in many cases of hypertension. It is released into the blood from the kidney, and cleaves from a blood glycoprotein a decapeptide known as angiotensin-I. Circulating angiotensin-I is cleaved in plasma, and in lung, kidney and other tissues to an octapeptide, angiotensin-II, which raises blood pressure both directly by causing arteriolar constriction and indirectly by stimulating release of the sodium-retaining hormone aldosterone from the adrenal gland and thus causing a rise in extracellular fluid volume. The latter effect is caused by angiotensin-II itself or a heptapeptide cleavage product angiotensin-III.

Inhibitors of renin have been described as useful in the treatment of hypertension.

A group of compounds has now been discovered, which inhibit the enzyme renin and therefore have potential blood pressure lowering activity, useful in the treatment of hypertension. Compounds having an associated mechanism of action have also been described as possessing anti-retroviral activity and the present compounds may therefore be of potential use in the treatment of diseases caused by retroviruses including human immunodeficiency virus (HIV-1 and 2) and HTLV I and II. They may also be of potential use in the treatment of other cardiovascular disorders, such as congestive heart failure, and have beneficial effects on learning and memory and mood elevation activity, of potential use in the treatment of CNS disorders such as Alzheimer's disease and depression; they may also be of potential use in the treatment of glaucoma.

Accordingly, the present invention provides a compound of formula (I), or a pharmaceutically acceptable salt thereof:

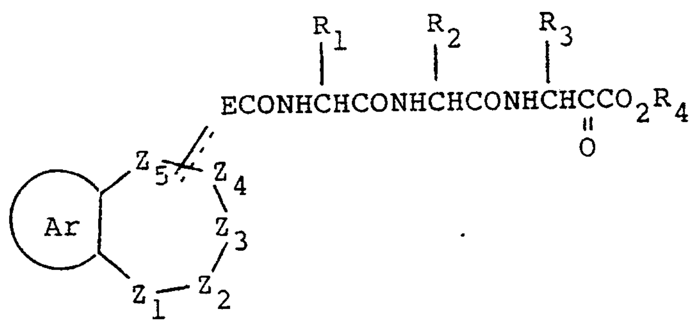

(I)

wherein
the Ar ring is of sub-formula (a) or (b):

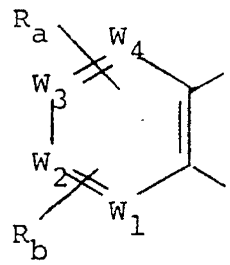

(a)

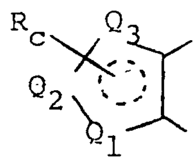

(b)

one or two of $W_1$, $W_2$, $W_3$ and $W_4$ is CH, N or NO (such that if two are NO then these are are $W_1$ and $W_4$), and the others are CH, $CR_a$ or $CR_b$;
one of $Q_1$, $Q_2$ and $Q_3$ is S and the other two are CH and $CR_c$;
either $Z_1$ and $Z_2$ are absent and $Z_3$, $Z_4$ and $Z_5$ and the carbon atoms to which $Z_3$ and $Z_5$ are attached, form a 5-membered non-aromatic heterocyclic ring; or
$Z_1$ is absent and $Z_2$, $Z_3$, $Z_4$, $Z_5$ and the carbon atoms to which $Z_2$ and $Z_5$ are attached, form a 6-membered non-aromatic heterocyclic ring; or
$Z_1$, $Z_2$, $Z_3$, $Z_4$ and $Z_5$ and the carbon atoms to which $Z_1$ and $Z_5$ are attached, form a 7-membered non-aromatic heterocyclic ring;
E is absent or is $(CH_2)_n$ or $CH(CH_2)_{n-1}$ wherein n is 1 to 4;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl or $C_{3-8}$ cycloalkyl or phenyl;
$R_4$ is $C_{1-6}$ alkyl, $C_{3-8}$ cycloalkyl, $C_{3-8}$ cycloalkyl-$C_{1-4}$ alkyl or a group of structure c), d), e) or f):

c) 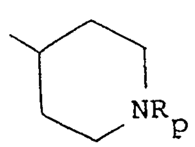

d) 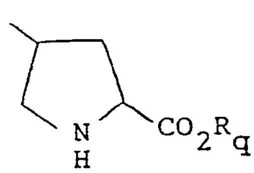

e) 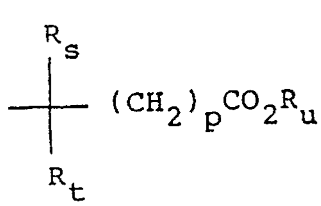

f) 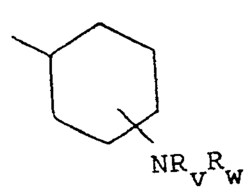

wherein
$R_p$, $R_q$, $R_s$, $R_t$, $R_u$, $R_v$ and $R_w$ are selected from hydrogen or $C_{1-6}$ alkyl; or $NR_vR_w$ is 1-imidazolyl; and

the dashed line represents an optional bond (when E is present).

When the Ar ring is of sub-formula (a)

Often, when one of $W_1$ to $W_4$ is N or NO, $W_3$ or $W_4$ is N or NO, usually $W_3$ is N when E is attached at $Z_5$, or $W_1$ is N when E is attached at $Z_5$.

When the Ar ring is of sub-formula (b)

Often $Q_1$ or $Q_3$ is S.

When $Z_1$ and $Z_2$ are absent:

Values of $Z_3$, $Z_4$ and $Z_5$ include wherein one of $Z_3$, $Z_4$ and $Z_5$ is attached to E and is N, CH or C (wherein the optional exocyclic bond is present); the second of $Z_3$, $Z_4$ and $Z_5$ is O, S, SO, $SO_2$ or NR wherein R is hydrogen or $C_{1-6}$ alkyl; and the third is O, S, SO, $SO_2$, NR, $CH_2$ or C=O provided that, one of $Z_3$ and $Z_4$, and one of $Z_4$ and $Z_5$, is $CH_2$, CH or C when the other is S or O, or is $CH_2$, CH, C, NR or N when the other is SO or $SO_2$; or $Z_4$ is SO, $SO_2$ or C=O when one of $Z_3$ and $Z_5$ is N and the other is O, S or NR; or $Z_3$ is CO, $Z_4$ is O and $Z_5$ is CH.

Examples of $Z_3$, $Z_4$ and $Z_5$ then include

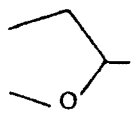 , 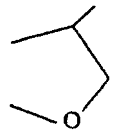 , 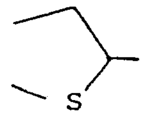 , 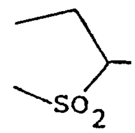 ,

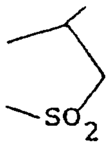 , 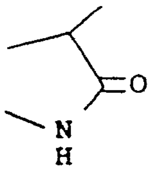 , 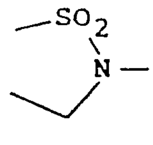 , 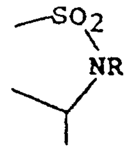 ,

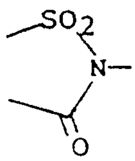 , 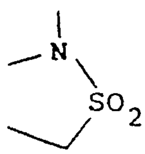 , 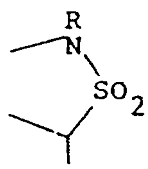 , 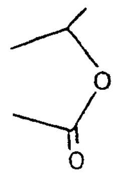 ,

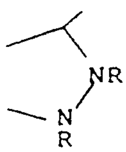 , 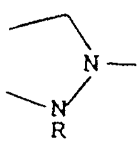 , 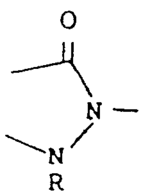 , 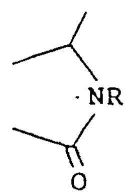 ,

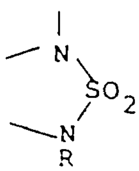 , 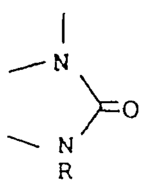 , 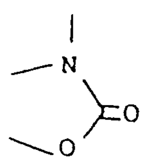 , 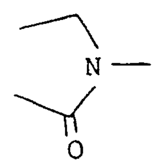

Preferably $Z_3$ is CO, $Z_4$ is N, $Z_5$ is CH and E is attached at $Z_4$.

<u>When $Z_1$ is absent:</u>

Values of $Z_2$, $Z_3$, $Z_4$ and $Z_5$ include wherein one of $Z_2$, $Z_3$, $Z_4$ and $Z_5$ is attached to E and is N, CH or C (wherein the optional exocyclic bond is present); another of $Z_2$, $Z_3$, $Z_4$ and $Z_5$ is O, S, SO, $SO_2$ or NR wherein R is hydrogen or $C_{1-6}$ alkyl; another of $Z_2$, $Z_3$, $Z_4$ and $Z_5$ is O, S, SO, $SO_2$, NR or $CH_2$; and the other of $Z_2$, $Z_3$, $Z_4$ and $Z_5$ is S, SO, $SO_2$, NR, $CH_2$ or C=O provided that, one of $Z_2$ and $Z_3$, one of $Z_3$ and $Z_4$, or one of $Z_4$ and $Z_5$ is $CH_2$, CH or C when the other is S or O, or is $CH_2$, CH, C, NR or N when the other is SO or $SO_2$; or $Z_3/Z_4$ is SO, $SO_2$ or C=O when one of $Z_2/Z_3$ and $Z_4/Z_5$ is N or NR and the other is O, S, N or NR; or $Z_2$-$Z_3$-$Z_4$-$Z_5$ is -NRCOCON< or >NCOCONR-; or $Z_2/Z_5$ is CO, $Z_3/Z_4$ is O and $Z_4,Z_5/Z_2,Z_3$ are CH and $CH_2$.

i.e. the following are excluded in $Z_2$ to $Z_5$:-

i) adjacent same heteroatoms (O, S, N) except N-N; but no N-N-N;

iii) $SO_x$-$SO_y$ or O-$S_y$ (x and y are 0-2);
iv) O-C-O, S-C-O, S-C-S;
v) CO-SO or CO-$SO_2$;
vi) two heteroatoms (O, S, N) separated by a Z when CO unless one of them is N;
vii) more than two of $Z_2$ to $Z_5$ is CO.

Examples of $Z_2$, $Z_3$, $Z_4$ and $Z_5$ then include

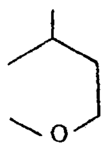 , 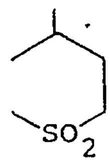 , 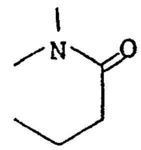 ,

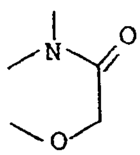 , 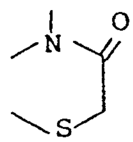 , 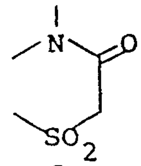 ,

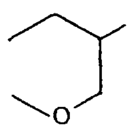 , 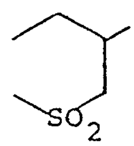 , 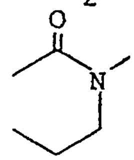 ,

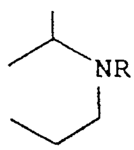 , 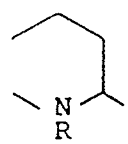 , 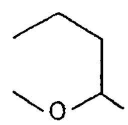 ,

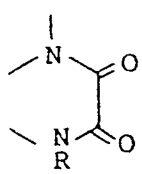 , 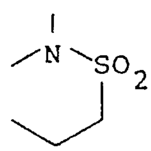 , 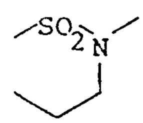 ,

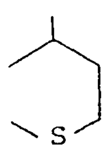 , 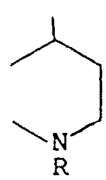 , 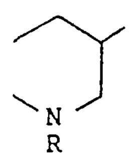 ,

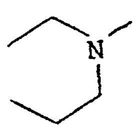

When $W_3$ is N and the other W are CH, $CR_a$ or $CR_b$, often $Z_2$ is S, $Z_3$ is $CH_2$, $Z_4$ is CO, $Z_5$ is N and E

is attached at $Z_5$.

When $Z_1$ and $Z_2$ are present

Heterocyclic ring heteroatoms for $Z_1$ to $Z_5$ are selected from oxygen, sulphur, SO, $SO_2$, nitrogen or N-alkyl. The ring may include unsaturation (C = C) or a carbon attached to exocyclic oxo (C = O).

Examples of $Z_1$, $Z_2$, $Z_3$, $Z_4$ and $Z_5$ then include

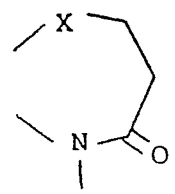 , 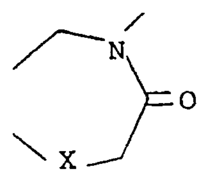 , 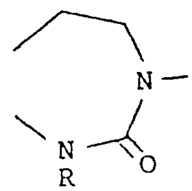 , 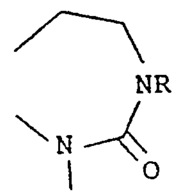

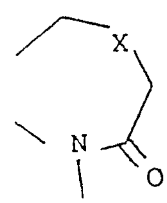 , 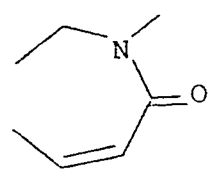 , 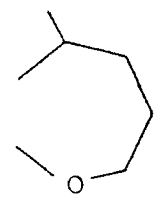

wherein X is O, S, SO, $SO_2$, NH, N-$C_{1-6}$ alkyl or $CH_2$.

Preferably E is attached at a nitrogen atom.

Suitable examples of $R_a$ and $R_b$ when one of $W_1$, $W_2$, $W_3$ and $W_4$ is N or NO, include a moiety selected from halogen, $C_{1-6}$ alkyl, $C_{1-6}$ alkoxy, cyano, CHO, $C_{1-6}$ alkyl substituted by optionally substituted amino or a group $NR_5R_6$, $CO_2R_5$, wherein $R_5$ is hydrogen, $C_{1-6}$ alkyl or optionally substituted benzyl and $R_6$ is hydrogen or $C_{1-6}$ alkyl.

When one of $R_a$ and $R_b$ is hydrogen and the other is a halogen substituent, the substituent is preferably in the 3-position relative to a ring nitrogen atom, when one or two of $W_1$ to $W_4$ is N or NO.

Suitable examples of substituents in optionally substituted amino groups in $R_a/R_b$ include one or two groups independently selected from $C_{1-6}$ alkyl or $C_{1-6}$ alkyl substituted by carboxy or $C_{1-6}$ alkoxycarbonyl or (when no W ring nitrogen atom), hydroxy or amino.

Suitable examples of $R_c$ in sub-formula (b), include a moiety selected from $C_{1-6}$ alkyl, optionally substituted amino, cyano, $C_{1-6}$ alkyl substituted by optionally substituted amino or $R_c$ is $CO_2R_5$ wherein $R_5$ is hydrogen, $C_{1-6}$ alkyl or optionally substituted benzyl.

Suitable examples of substituents in optionally substituted amino groups in $R_a/R_b/R_c$ then include one or two groups independently selected from $C_{1-6}$ alkyl or $C_{1-6}$ alkyl substituted by carboxy or $C_{1-6}$ alkoxycarbonyl.

Alternatively, $NR_5R_6$ or other substituted amino groups in $R_a/R_b/R_c$ may be pyrrolidinyl, piperidinyl, morpholinyl or piperazinyl optionally N-substituted by $C_{1-6}$ alkyl, or as an N-oxide thereof.

$R_c$ is preferably hydrogen.

Preferably one of $R_a$ and $R_b$ is hydrogen and the other is $CH_2NH_2$, $CO_2H$, $CH_2NHCH_2CO_2H$ or (when no W ring nitrogen atom), $SO_2CH_2CO_2H$.

Suitable values of $R_9$ and $R_{11}$ when aryl include phenyl or naphthyl and when heteroaryl, include a 5- or 6- membered monocyclic or 9- or 10- membered bicyclic of which 5- or 6-membered monocyclic heteroaryl is preferred. In addition, 5- or 6-membered monocyclic or 9- or 10-membered bicyclic heteroaryl preferably contains one, two or three heteroatoms which are selected from the class of oxygen, nitrogen and sulphur and which, in the case of there being more than one heteroatom, are the same or different. Examples of 5- or 6-membered monocyclic heteroaryl containing one, two or three heteroatoms which are selected from the class of oxygen, nitrogen and sulphur include furyl, thienyl, pyrryl, oxazolyl, thiazolyl, imidazolyl and thiadiazolyl, and pyridyl, pyridazyl, pyrimidyl, pyrazolyl and triazolyl. Preferred examples of such groups include furanyl, thienyl, pyrryl and pyridyl, in particular 2- and 3-furanyl, 2- and 3-pyrryl, 2- and 3-thienyl, and 2-, 3- and 4-pyridyl. Examples of 9- or 10-membered bicyclic heteroaryl containing one, two

or three heteroatoms which are selected from the class of oxygen, nitrogen and sulphur include benzofuranyl, benzothienyl, indolyl and indazolyl, quinolyl and isoquinolyl, and quinazolyl. Examples of such groups include 2- and 3-benzofuranyl, 2- and 3-benzothienyl, and 2- and 3-indolyl, and 2- and 3-quinolyl and, for $R_{11}$, 4-benzimidazolyl.

Suitable examples of groups or atoms for optional substitution of $R_5$ when benzyl and $R_9$ and $R_{11}$ include one, two or three substituents independently selected from $C_{1-4}$ alkyl, $C_{1-4}$ alkoxy, halo (such as fluoro, chloro, bromo), hydroxy, nitro, and cyano.

Preferred examples of $R_9$ include phenyl and naphthyl, and preferred examples of $R_{11}$ when aryl or heteroaryl include phenyl, imidazol-4-yl and -2-yl, pyrazol-1-yl and 4-methylpyrazol-1-yl.

Preferably the amino acid residue containing $R_2$ is Leu, β-pyrazolylalanine or His.

Abbreviations which may be used herein are as follows:

| Amino Acid Residue | Abbreviations |
|---|---|
| histidine | His |
| isoleucine | Ile |
| leucine | Leu |
| 1-naphthylalanine | NAla |
| norleucine | Nle |
| phenylalanine | Phe |

The α-amino acid components are in the (S)-configuration (or L-form).

In a particular aspect, the present invention provides a compound of formula (IA) or a pharmaceutically acceptable salt thereof:

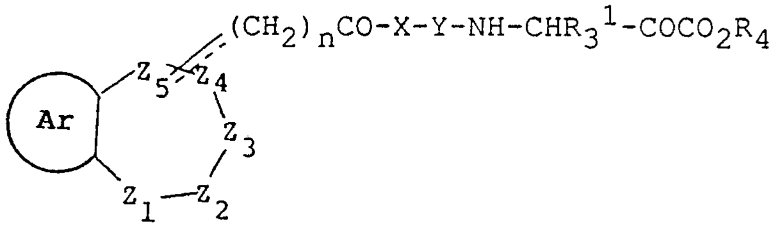

(IA)

wherein

X is Phe;

Y is Leu or His;

$R_3{}^1$ is cyclohexylmethyl; and

the remaining variables are as defined in formula (I).

Suitable values for alkyl groups in $R_a$, $R_b$, R and $R_2$ to $R_{12}$ in formulae (I) and (IA) include methyl, ethyl, n- and iso-propyl, n-, sec-, iso- and tert-butyl, n-, iso-, sec-, tert- and neo-pentyl. $C_{3-8}$ cycloalkyl groups are cyclopropyl, cyclobutyl, cyclopentyl, cyclohexyl, cycloheptyl and cyclooctyl.

Suitable values for $R_a$ and $R_b$ halo include fluoro, chloro, bromo and iodo, preferably chloro or bromo.

Preferably the carbon atom in $R_4$ which is adjacent $CO_2$, is either a secondary or tertiary carbon atom.

Preferably n is 1, 2, 3 or 4.

E is preferably $(CH_2)_n$ attached at a $Z_1$ to $Z_5$ nitrogen atom, when the $W_1$ to $W_4$ Ar ring is phenyl.

When $Z_1$ is absent, E is favourably attached at the 4-position with respect to $Z_2$ when n = 2, and at the 3-position when n>2. When $Z_1$ and $Z_2$ are absent, E is preferably at the 3-position with respect to $Z_3$ when n = 2, and at the 2-position when n>2. When $Z_1$ and $Z_2$ are present, E is usually attached at a nitrogen atom or at $Z_1$ or $Z_5$ when a carbon atom. E is preferably attached at a nitrogen atom.

Pharmaceutically acceptable salts include acid addition salts which may be, for example, salts with inorganic acids such, for example, as hydrochloric acid, hydrobromic acid, orthophosphoric acid or sulphuric acid, or organic acids such, for example, as methanesulphonic acid, toluenesulphonic acid, acetic acid, trifluoroacetic acid, propionic acid, lactic acid, citric acid, tartaric acid, fumaric acid, malic acid, succinic acid, salicylic acid or acetylsalicylic acid.

Phamaceutically acceptable salts may also include alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)amine or tris-(2-hydroxyethyl) amine.

It will be appreciated that the compounds of the invention may exist as solvates, such as hydrates, and these are included whenever, a compound of formula (I) or a salt thereof, is herein referred to.

The compounds of formula (I) wherein E is attached to $Z_1$(when present)/$Z_2$(when present)/$Z_3$/$Z_4$/$Z_5$ when CH, and (IA) have at least one asymmetric centre in addition to those attached to $R_1$ and $R_2$ (in X and Y) and $R_3$, and are therefore capable of existing in more than one stereoisomeric form. The invention extends to each of these forms and to mixtures thereof. Preferably the configuration at the carbon atoms bearing $R_1$, $R_2$ and $R_3$ is the (S)-configuration, although it will be appreciated that epimerisation may readily occur at the $R_3$ carbon atom.

It will be appreciated that, when the optional bond depicted in formula (I) is present, the compounds are capable of existing in E and Z (trans and cis) forms. The invention extends to each of these forms and to mixtures thereof.

The compounds of the invention are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

A compound of the invention may be prepared by those methods known in the art for the synthesis of compounds of analogous structure, such as peptides, and in this regard reference is made, by way of illustration only, to the literature reference: S.R. Pettit, "Synthetic Peptides", (Elsevier Scientific Publishing Co. 1976).

The present invention also provides a compound of the present invention which has been prepared synthetically.

A compound of the present invention may, for example, be formed by the sequential coupling of appropriate amino acids with the acid of formula (II):

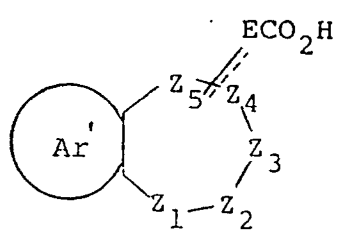

(II)

wherein Ar' is Ar as defined in formula (I), but substituted by $R_a'$, $R_b'$ in sub-formula (a) and by $R_c'$ in sub-formula (b), wherein $R_a'$, $R_b'$ and $R_c'$ are $R_a$, $R_b$ and $R_c$ respectively or groups or atoms convertible thereto; or by the initial preparation and subsequent coupling of peptide subunits with the acid of formula (II), the subunits themselves prepared in stepwise manner; in either case classical solution chemistry methods analogous to those used in peptide synthesis, may be employed.

he coupling reactions may be effected by, for example, activating the reacting carboxyl group of the acid of formula (II) or amino acid, and reacting this with the amino group of the substrate unit. Details of suitable, optional activating and protecting (masking) groups and of suitable reaction conditions (for the coupling reactions and for the introduction and removal of protecting groups) giving, preferably, the minimum of racemisation, may be found in the above-referenced literature.

Accordingly, the present invention further provides a process for the preparation of a compound of formula (I) or a pharmaceutically acceptable salt thereof which comprises reacting a reagent of the formula

(III):

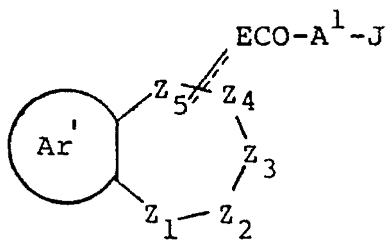

(III)

wherein Ar' is as defined in formula (II), $A^1$ is absent or represents an appropriate amino acid or dipeptide unit; J is OH or a leaving group; and the remaining variables are as hereinbefore defined; with a reagent of the formula (IV):

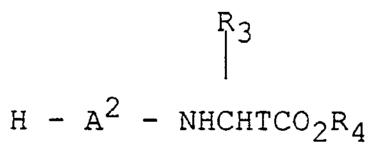

(IV)

wherein
$A^2$ is absent or represents an appropriate amino acid or dipeptide unit, such that $A^1$ + $A^2$ is $-NHCHR_1CONHCHR_2CO-$; T is an optionally protected carbonyl group; and the remaining variables are as hereinbefore defined; and thereafter, if desired or necessary deprotecting (T or within $A^1$ or $A^2$) of the products, and/or converting $Z_1$, $Z_2$, $Z_3$, $Z_4$ or $Z_5$ to other $Z_1$, $Z_2$, $Z_3$, $Z_4$ or $Z_5$, $R_a'/R_b'/R_c'$ in Ar' to $R_a$, $R_b$, and/or $R_c$ and/or $W_1$, $W_2$, $W_3$ or $W_4$ in Ar' when N to NO; and/or forming a pharmaceutically acceptable salt thereof.

Suitable examples of J when a leaving group include halo, such as chloro or bromo, and other suitable groups which are displaceable by an amino nucleophile, such as $C_{1-6}$ alkoxycarbonyloxy.

It is generally preferred, especially when $A^1$ is not absent, however, that J is OH, and a suitable coupling reagent or dehydrating catalyst is used to effect the reaction, such as N,N-dicyclohexylcarbodiimide and those described in the Descriptions and Examples hereinafter.

Suitable protected derivatives for T include the thioketal derivative, which may be deprotected as described in Example 1 hereinafter.

Deprotection of $A^1$ and/or $A^2$ takes place conventionally, in accordance with the particular protecting group(s) employed.

Pharmaceutically acceptable salts may be formed conventionally.

$Z_1$, $Z_2$, $Z_3$ or $Z_4$ when S or SO may be converted to SO or $SO_2$ respectively (or S to $SO_2$) by conventional oxidation methods, preferably in a non-aqueous solvent, such as a chlorinated hydrocarbon, in the presence of an organic peracid, such as 3-chloroperoxybenzoic acid, or in water in the presence of a soluble strong inorganic oxidant, such as an alkali metal permanganate or with aqueous hydrogen peroxide.

It will be apparent that compounds of the formula (I) containing an an Ar' ring wherein the $R_a'/R_b'/R_c'$ group which is other than $R_a/R_b/R_c$ and which is convertible to an $R_a/R_b/R_c$ group, are useful novel intermediates. A number of such conversions is possible, not only for the final product compounds of formula (I) when $R_a'/R_b'/R_c'$ are other than $R_a/R_b/R_c$, but also within $R_a/R_b/R_c$, and also for their intermediates as follows:

(i) a hydrogen substituent is convertible to a nitro substituent by nitration;
(ii) a nitro substituent is convertible to an amino substituent by reduction;
(iii) a $C_{1-7}$ acylamino substituent is convertible to an amino substituent by deacylation;
(iv) an amino substituent is convertible to a $C_{1-7}$ acylamino substituent by acylation with a carboxylic

acid derivative;

(v) a hydrogen substituent is convertible to a halogen substituent by halogenation;

(vi) an $C_{1-6}$ alkylthio or a $C_{1-6}$ alkylsulphinyl substituent is convertible to a $C_{1-6}$ alkylsulphinyl or $C_{1-6}$ alkylsulphonyl substituent respectively, by oxidation;

(vii) an amino, aminosulphonyl, or $NHCONH_2$ substituent is convertible to a corresponding substituent which is substituted by one or two alkyl groups, by N-alkylation;

(viii) an amino substituent is convertible to a group $NHCONH_2$, by reaction with potassium cyanate and acid;

(ix) a hydrogen substituent is convertible to an aminosulphonyl substituent by treatment with $ClSO_3H$ followed by $HNR_5R_6$;

(x) a cyano substituent may be converted to an aminomethyl substituent by reduction;

(xi) a bromo substituent may be converted to a cyano substituent by reaction with copper (I) cyanide.

Conversions (i) to (xi) are only exemplary and are not exhaustive of the possibilities. It will be appreciated that it is often desirable to carry out these conversions at an earlier stage, in the intermediate acid of formula (II), especially in regard to conversion (iii).

In regard to (i), nitration is carried out in accordance with known procedures.

In regard to (ii), the reduction is carried out with a reagent suitable for reducing nitroanisole to aminoanisole.

In regard to (iii), deacylation is carried out by treatment with a base, such as an alkali metal hydroxide.

In regard to (iv), the acylation is carried out with an acylating agent, such as the corresponding acid or acid chloride. Formylation is carried out with the free acid.

In regard to (v), halogenation is carried out with conventional halogenating agents.

In regard to (vi), oxidation is carried out at below ambient temperatures in a non-aqueous solvent, such as a chlorinated hydrocarbon, in the presence of an organic peracid, such as 3-chloroperoxybenzoic acid, or in water in the presence of a soluble strong inorganic oxidant, such as an alkali metal permanganate or with aqueous hydrogen peroxide.

In regard to (vii), alkylation is carried out with a corresponding alkylating agent such as the chloride or bromide under conventional conditions.

In regard to (viii), conversion to a ureido derivative is carried out by reaction with potassium cyanate in acidic methanol, at ambient temperature.

In regard to (ix), the mixing with $ClSO_3H$ takes place at low temperatures around 0° C and allowed to warm to ambient temperature. The subsequent reaction with the amine takes place at ambient temperature, in a solvent such as ethanol.

In regard to (x), the reduction takes place by reaction with sodium borohydride/cobalt chloride, or platinum oxide/hydrogen.

In regard to (xi), the reaction takes place under conventional conditions.

Compounds of the general formulae (II) and (III) may themselves be prepared by standard techniques analogous to those described above.

The acids of formula (II) are either known compounds or are prepared by analogous methods to these and for structurally similar known compounds.

The preparation of the amino acid unit of formula (V):

$$H_2N\text{-}CHR_3{}^1T\text{-}CO_2H \qquad (V)$$

as an appropriate derivative thereof, is described in Descriptions 1 and 2 hereinafter.

It will be appreciated that protected forms of compounds of the present invention are novel intermediates and form an aspect of the invention.

Particularly suitable methods of preparation of the compounds of the present invention are as described in the Descriptions and Examples hereinafter. The couplings may be carried out sequentially beginning by coupling the acid of formula (II) with, for example, phenylalanine or 1-naphthylalanine, followed by coupling with Y, for example, leucine or histidine, and finally, coupling with the amino acid of formula (V) . In an alternative aspect, however, either the acid of formula (II) is coupled with a tripeptide unit formed between the amino acid of formula (V), leucine, histidine or other $R_2$ containing amino acid; and phenylalanine or naphthylalanine; or alternatively the acid of formula (II) is coupled with phenylalanine or naphthylalanine and this is coupled with a dipeptide unit formed between the $R_2$ containing amino acid and the amino acid of formula (V).

As mentioned previously the compounds of the invention have been found to be renin inhibitors, and therefore they are of potential use in the treatment of hypertension. They may also be of potential use in the other diseases and disorders hereinbefore referred to.

The present invention also provides a pharmaceutical composition which comprises a compound of formula (I) or a pharmaceutically acceptable salt thereof, and a pharmaceutically acceptable carrier. In particular, the present invention provides an anti-hypertensive pharmaceutical composition which comprises an anti-hypertensive effective amount of a compound of formula (I) or a pharmaceutically acceptable salt thereof, and a pharmaceutically acceptable carrier.

The compositions are preferably adapted for oral administration. However, they may be adapted for other modes of administration, for example parenteral administration for patients suffering from heart failure. Other alternative modes of administration include sublingual or transdermal administration, or for example, eye drop compositions, for treatment of glaucoma.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

For transdermal administration, formulations suitable for topical use may be employed, optionally containing penetration enhancers.

The compositions may contain from 0.1% to 99% by weight, preferably from 10-60% by weight, of the active material, depending on the method of administration.

The present invention further provides a method of prophylaxis or treatment of hypertension in mammals including man, which comprises administering to the suffering mammal an anti-hypertensively effective amount of a compound of formula (I) or a pharmaceutically acceptable salt thereof.

An effective amount will depend on the relative efficacy of the compounds of the present invention, the severity of the hypertension being treated and the weight of the sufferer.

However, a unit dose form of a composition of the invention may contain from 0.1 to 500 mg of a compound of the invention and more usually from 1 to 100 mg, for example 2 to 50 mg such as 2, 3, 4, 5, 10, 20 or 30mg. Such compositions may be administered from 1 to 6 times a day, more usually from 2 to 4 times a day, in a manner such that the daily dose is from 1 to 1000mg for a 70 kg human adult and more

particularly from 5 to 500 mg.

No toxicological effects are indicated at the aforementioned dosage ranges.

The present invention further provides a compound of formula (I) or a pharmaceutically acceptable salt thereof for use in the treatment or prophylaxis of hypertension.

The following Descriptions relate to the preparation of intermediates and the following Examples relate to the preparation of compounds of formula (I).

The following notations may be employed:-

| | |
|---|---|
| Benzyloxycarbonyl | CBZ |
| *tert*-Butyloxycarbonyl | BOC |

Description 1

a) 1,3-Dithiane-2-carboxylic acid

2-Carboethoxy-1,3-dithiane (18.64g) was dissolved in tetrahydrofuran (THF) (600 ml), and sodium hydroxide (1M aqueous solution, 97.1 ml) was added. The mixture was stirred for 35 h, evaporated *in vacuo*, and partitioned between ethyl acetate (300 ml) and water (300 ml). The aqueous portion was acidified (5M hydrochloric acid) and extracted with ether. The extracts were dried ($Na_2SO_4$) and evaporated *in vacuo*, giving the title compound (16.4g) as an off-white solid.

NMR (δ) ($CDCl_3$): 2.0-2.9 (4H,m), 3.2-3.8 (2H,m), 4.2 (1H,s), 10.3 (1H,s).

b) Isopropyl 1,3-dithiane-2-carboxylate

1,3-Dithiane-2-carboxylic acid (16.0g) was dissolved in isopropanol (400 ml), and concentrated sulphuric acid (15 ml) was added. The mixture was stirred at 40°C for 43 h, evaporated *in vacuo*, partitioned between ethyl acetate (400 ml) and saturated sodium hydrogen carbonate solution (100 ml), and separated. The organic portion was washed with brine, dried ($Na_2SO_4$) and evaporated *in vacuo*, giving the title compound (15.8g) as a pale yellow oil.

NMR (δ) ($CDCl_3$): 1.25 (6H,d), 1.9-2.8 (4H,m), 3.1-3.8 (2H,m), 4.1 (1H,s), 5.05 (1H,m).

Description 2

a) Cyclohexylacetaldehyde

Methyl cyclohexylacetate (18.0g) was stirred under nitrogen in dry toluene (200 ml) as diisobutylaluminium hydride (1.5M solution in toluene, 200 ml) was added over 1.5 h, maintaining temperature at <-63°C throughout. The mixture was stirred at <-65°C for 1 h, when methanol (12.1 ml) was added over 15 min. After stirring at <-60°C for 15 min, the mixture was warmed to -5°C, when saturated potassium sodium tartrate solution (450 ml) was added over 45 min. This mixture was extracted with ethyl acetate (1100 ml), and the extract was washed with water, dried ($Na_2SO_4$), and evaporated *in vacuo*, giving the title compound (12.66g) as a light yellow oil, contaminated with ca.40% cyclohexylethanol (NMR).

NMR (δ) ($CDCl_3$): 0.7-2.1 (m), 2.3 (m, $CH_2CHO$), 3.6 (m, $CH_2OH$ contaminant) 9.7 (m, CHO).

b) N-CBZ-2-cyclohexyl-1-(toluene-p-sulphonyl)ethylamine

Benzyl carbamate (10.38g), sodium toluene-p-sulphinate hydrate (16.10g), cyclohexylacetaldehyde (contaminated as noted in Description 2(a)) (12.77g) and formic acid (16.0 ml) were stirred at reflux in water (160 ml) for 3h. After cooling overnight, the solid was filtered off, dissolved in dichloromethane, dried ($Na_2SO_4$) and evaporated *in vacuo* to give the title compound (26.84g) as a white solid.

NMR (δ) ($CDCl_3$): 0.7-2.2 (13H,m), 2.35 (3H,s), 4.9 (2H,s), 5.0-5.7 (2H,s), 7.0-7.4 (7H,m), 7.75 (2H,d).

c) Isopropyl 2-(1-(CBZ-amino)-2-cyclohexylethyl)-1,3-dithiane-2-carboxylate

To a solution of diisopropylamine (10.6 ml) in dry THF (580 ml) under nitrogen at <-20 °C was added n-butyllithium (1.6M solution in hexanes, 47.4 ml). After 20 min at 0 °C, the solution was cooled to <-67 °C, at which temperature isopropyl 1,3-dithiane-2-carboxylate (Description 1(b)) (15.6g) was added in dry THF (45 ml). After 30 min at <-70 °C, N-CBZ-2-cyclohexyl-1-(toluene-p-sulphonyl)ethylamine (26.7g) was added in dry THF (125 ml) over 15 min. The mixture was stirred at <-70 °C for 1.5h, and warmed to -30 °C, when 10% aqueous citric acid (600 ml) was added.

The organic layer was separated off, diluted with ethyl acetate, washed with water, dried ($Na_2SO_4$) and evaporated in vacuo to give a yellow oil. Chromatography on silica gel using ether/hexane (10-30% ether, gradient) gave the title compound (12.46g) as a colourless oil which slowly solidified on standing.

NMR (δ) ($CDCl_3$): 0.7-1.75 (18H,m), 1.8-2.2 (3H,m), 2.7 (2H,m), 3.15 (2H,m), 4.4 (1H,td), 5.0-5.4 (4H,m), 7.35 (5H,m).

Description 3

a) 2-(2,3-Dihydro-1,1-dioxobenzothiophene-3-yl)malonic acid

To a solution of diethyl 2-(2,3-dihydro-1,1-dioxobenzothiophene-3-yl)malonate (11.6g)* in ethanol (150 ml) was added sodium hydroxide (4g) in water (30 ml). The mixture was stirred at reflux for 4h, cooled and the ethanol was removed under reduced pressure. The residue was diluted with water (20 ml) and was extracted with ether (3x100 ml) and dichloromethane. The aqueous layer was acidified to pH 1 with 5M hydrochloric acid and was extracted with ether (3x80 ml) and dichloromethane (3x80 ml). The combined extracts (3x ether + 3x dichloromethane) were dried over sodium sulphate and the solvent was removed under reduced pressure to give the title compound (6.2g).

NMR (δ) ($DMSOd_6$): 3.6-3.9 (2H, m), 4.1-4.3 (2H, m), 7.5-7.9 (4H, m), 13.2 (2H, b).

b) (2,3-Dihydro-1,1-dioxobenzothiophene-3-yl)acetic acid

2-(2,3-Dihydro-1,1-dioxobenzothiophene-3-yl)malonic acid (1.9g) was heated in xylene at reflux for 3h. The mixture was cooled and extracted with saturated sodium hydrogen carbonate (2x50 ml). The combined aqueous extracts were washed with ether (50 ml) and acidified to pH 2 with 5M hydrochloric acid. The mixture was extracted with ether (2x80 ml) and dichloromethane (2x80 ml). The combined extracts were dried ($Na_2SO_4$) and the solvent was removed under reduced pressure to give the title compound (1.4g).

NMR (δ) ($DMSOd_6$): 2.6-2.8 (1H, m), 2.9-3.1 (1N, m), 3.4-3.6 (1H, m), 3.8-4.0 (2H, m), 7.5-7.9 (4H, m), 12.6 (1H, b).

c) (2,3-Dihydro-1,1-dioxobenzothiophene-3-yl)acetyl-Phe-Leu-OH

This material was formed from (2,3-dihydro-1,1-dioxobenzothiophene-3-yl)acetic acid (0.50g) and Phe-Leu methyl ester.$CH_3CO_2H$ (0.65g), following the coupling procedure in Description 3(d). The intermediate product was then dissolved in ethanol (10 ml), and a slight excess of 10% aqueous sodium hydroxide was added. After 4h, the mixture was diluted with water, washed with chloroform, acidified (5M hydrochloric acid) and extracted with ethyl acetate. The extract was dried ($Na_2SO_4$) and evaporated in vacuo to give the title compound (0.50g).

NMR (δ) ($CDCl_3$): 0.6-2.0 (9H,m), 2.0-5.1 (9H,m), 6.6-8.1 (11H,m), 9.4 (1H,b).

d) Isopropyl 2-(1-((2,3-dihydro-1,1-dioxobenzothiophene-3-yl)acetyl-Phe-Leu-amino)-2-cyclohexylethyl)-1,3-dithiane-2-carboxylate

Isopropyl 2-(1-(CBZ-amino)-2-cyclohexylethyl)-1,3-dithiane-2-carboxylate (Description 2(c)) (0.50g) was dissolved in glacial acetic acid (4 ml), and hydrobromic acid (45% in glacial acetic acid, 2 ml) was added with stirring. The mixture was left to stand for 1.5h, and evaporated in vacuo to a light yellow foam. To this were added (2,3-dihydro-1,1-dioxobenzothiophene-3-yl)acetyl-Phe-Leu-OH (0.52g), 1-hydroxybenzotriazole (HOBT) (0.16g) and dry dimethylformamide (DMF) (5 ml). The solution was stirred at 0 °C, and N,N-diisopropylethylamine (DIPEA) (0.37 ml) and 1-(3- dimethylaminopropyl)-3-ethylcarbodiimide. HCl (DEC) (0.21g) were added. The mixture was stirred overnight, warming to ambient temperature, diluted with ethyl acetate, washed with 5% aqueous citric acid, water, 5% aqueous sodium hydrogen carbonate, and brine, dried ($Na_2SO_4$) and evaporated in vacuo. Chromatography on silica gel using methanol/chloroform (0-2% methanol, gradient) gave the title compound (0.82g) as a white foam.

NMR (δ) ($CDCl_3$): 0.7-1.05 (8H,m), 1.05-2.2 (22H,m), 2.5-2.9 (4.5H,m), 2.9-3.4 (5H,m), 3.6 (0.5H,m), 4.05 (1H,m), 4.45 (1H,m), 4.6-4.9 (2H,m), 5.1 (1H,m), 6.3-6.75 (2H,m), 7.1-8.0 (10H,m).

Description 4

(a) Isopropyl 2-(1-(BOC-Phe-Leu)amino-2-cyclohexylethyl)-1,3-dithiane-2-carboxylate

This material was formed from isopropyl 2-(1-CBZ-amino)-2-cyclohexylethyl)-1, 3-dithiane-2-carboxylate (Description 2(c)) (2.07g) and BOC-Phe-Leu-OH (1.85g), following the procedure of Description 3(d). The crude product was chromatographed on silica gel using methanol/chloroform (0-1% methanol, gradient). This gave the title compound (3.03g).

NMR (δ) ($CDCl_3$): 0.7-1.0 (8H, m), 1.05-1.4 (20H, m), 1.45-2.15 (11H, m), 2.75 (2H, m), 2.95-3.25 (4H, m), 4.4 (2H, m), 4.65 (1H, m), 4.9 (0.5H, m), 5.1 (1H, m), 6.2 (0.5H, d), 6.5 (1H, m), 7.15-7.4 (6H, m).

(b) Isopropyl 2-(1-(Phe-Leu)amino-2-cyclohexylethyl)-1,3-dithiane-2-carboxylate.TFA

Isopropyl 2-(1-BOC-Phe-Leu)amino-2-cyclohexylethyl)-1,3-dithiane-2-carboxylate (2.90g) was dissolved with stirring in trifluoracetic acid (TFA) (20 ml) and dichloromethane (DCM) (20 ml) at 0° C. After 30 min, solvent was removed in vacuo, the residue dissolved in water (40 ml) and freeze-dried to give the title compound (2.8g) as a yellow foam.

NMR (δ) ($CD_3OD$): 0.75-1.05 (5H, m), 1.1-1.5 (12H, m), 1.5-2.4 (13H, m), 2.7 (2H, m), 2.85-3.15 (2H, m), 3.3-3.45 (2H, m), 3.9-4.7 (2H, m), 4.9-5.2 (2H, m), 7.35 (6H, m).

Description 5

(a) tert-Butyl (4-cyano-2-nitrophenoxy) acetate

4-Cyano-2-nitrophenol* (3.12g), sodium hydroxide (1.22g), benzyltributylammonium bromide (0.34g) and tert-butyl bromoacetate (5.6 ml) were stirred vigorously at reflux in a mixture of water (100 ml) and 1,2-dichloroethane (100 ml) for 16h. The mixture was cooled, acidified (5M hydrochloric acid) and separated. The aqueous portion was extracted with dichldoromethane, and the combined organics were dried ($Na_2SO_4$) and evaporated in vacuo. Chromatography on silica gel using ethyl acetate/petroleum ether (bp 60-80° C) (10-40% ethyl acetate, gradient) gave the title compound (4.26g) as a cream solid, mp 121-3° C.

NMR (δ) ($CDCl_3$): 1.5 (9H, s), 4.75 (2H, s), 7.05 (1H, d), 7.8 (1H, dd), 8.2 (1H, d).

(b) 6-Cyano-2,3-dihydro-3-oxo-4H-1,4-benzoxazine

tert-Butyl (4-cyano-2-nitrophenoxy)acetate (5.33g) was stirred in glacial acetic acid (100 ml) at 40° C as iron (electrolytic, 4x3.2g portions) was added over 2h. After a further 1h, the mixture was evaporated in vacuo, and the residue was suspended in water (500 ml). Filtration and drying in vacuo then gave the title compound (2.74g) as a grey solid.

NMR (δ) ($DMSOd_6$): 4.75 (2H, s), 7.0-7.5 (3H, m), 12.0 (1H, b).

(c) 6-(BOC-aminomethyl)-2,3-dihydro-3-oxo-4H-1,4-benzoxazine

6-Cyano-2,3-dihydro-3-oxo-4H-1,4-benzoxazine (0.18g) was hydrogenated over platinum dioxide (0.05g) in ethyl acetate (25 ml) containing di-tert-butyldicarbonate (0.23g) for 70h. Catalyst was filtered off, and the filtrate was evaporated. The crude product was chromatographed on silica gel using ethyl acetate/petroleum ether (bp 60-80° C) (50-100% ethyl acetate, gradient). This gave the title compound (0.18g) as a white solid.

NMR (δ) ($DMSOd_6$): 1.35 (9H, s), 4.0 (2H, d), 4.5 (2H, s), 6.75 (2H, m), 6.85 (1H, d), 7.35 (1H, t), 10.7 (1H, s).

(d) Methyl 3-(6-(BOC-aminomethyl)-2,3-dihydro-3-oxo-4H-1, 4-benzoxazin-4-yl)propanoate

6-(BOC-aminomethyl)-2,3-dihydro-3-oxo-4H-1,4- benzoxazine (0.22g), anhydrous potassium carbonate (0.16g) and methyl acrylate (0.07 ml) were stirred in dry DMF for 70h. The mixture was diluted with ethyl

* Proc. Roy. Soc. (London) SerB, Vol.133, 30 (1946).

acetate (50 ml), washed with water and brine, dried ($Na_2SO_4$) and evaporated in vacuo to give the title compound (0.27g) as a yellow solid.

NMR (δ) ($CDCl_3$): 1.45 (9H, s), 2.7 (2H, t), 3.7 (3H, s), 4.25 (4H, m), 4.6 (2H, s), 4.9 (1H, b), 6.95 (3H, m).

(e) 3-(6-(BOC-aminomethyl)-2,3-dihydro-3-oxo-4H-1,4-benzoxazin-4-yl)propanoic acid

Methyl 3-(6-(BOC-aminomethyl)-2,3-dihydro-3-oxo-4H-1,4-benzoxazin-4-yl)propanoate (0.26g) and 10% aqueous sodium hydroxide (0.4 ml) were dissolved in ethanol and stirred for 2h. The mixture was diluted with water, washed with ethyl acetate, acidified (5M hydrochloric acid), and extracted with chloroform. The extract was dried ($Na_2SO_4$) and evaporated in vacuo to give the title compound (0.26g) as a white solid.

NMR (δ) ($CDCl_3$): 1.45 (9H, s), 2.75 (2H, t), 4.25 (2H, m), 4.6 (2H, s), 5.05 (1H, b), 6.9 (2H, m),7.05 (1H, b).

(f) Isopropyl 2-(1-(3-(6-(BOC-aminomethyl)-2,3-dihydro-3-oxo-4H-1,4-benzoxazin-4-yl)propanoyl-Phe-Leu)-amino-2-cyclohexylethyl)-1,3-dithiane-2-carboxylate

3-(6-(BOC-aminomethyl)-2, 3-dihydro-3-oxo-4H-1,4-benzoxazin-4-yl)propanoic acid (0.30g) and HOBT (0.12g) were stirred in DMF (3 ml) in ice. DEC (0.17g) was added and the mixture was stirred for 15 min. DIPEA (0.3 ml) and isopropyl 2-(1-(Phe-Leu)amino-2-cyclohexylethyl)-1,3-dithiane-2-carboxylate.TFA (Description 4(b)) (0.56g) were added. The mixture was stirred for 16h, warming to ambient temperature, diluted with ethyl acetate, washed with 5% aqueous citric acid, water, 5% aqueous sodium hydrogen carbonate and brine, dried ($Na_2SO_4$) and evaporated in vacuo. Chromatography on silica gel using methanol/chloroform (0-5% methanol, gradient) gave the title compound (0.57g) as a white foam.

NMR (δ) ($CDCl_3$): 0.7-1.0 (5H, m), 1.05-1.3 (3H, m), 1.3-1.4 (6H, m), 1.4-1.5 (9H, m), 1.5-2.2 (16H, m), 2.55 (2H, m), 2.7 (2H, m), 3.1 (4H, m), 4.05-4.3 (4H, m), 4.5 (2H, m), 4.6-4.75 (2H, m), 5.1 (2H, m), 6.45-6.7 (2H, m), 6.9 (2H, m), 7.0 (1H, m), 7.1-7.45 (7H, m).

Description 6

(a) t-Butyl 3-(2,3-dihydro-3-oxo-4H-pyrido[4,3-b]-1,4-thiazin-4-yl) propanoate

This material was prepared from 2,3-dihydro-3-oxo-4H-pyrido[4,3-b]-1,4-thiazine *(4.0g) and t-butyl acrylate (3.5 ml) following the procedure of Description 5 (d). Chromatography on silica gel using methanol/chloroform (0-4% methanbol, gradient) gave the title compound (6.0g) as a cream solid.

NMR (δ) ($CDCl_3$): 1.4 (9H, s), 2.65 (2H, t), 3.45 (2H, s), 4.3 (2H, t), 7.3 (1H, dd), 8.2 (1H, dd), 8.45 (1H, s).

(b) 3-(2,3-Dihydro-3-oxo-4H-pyrido [4,3-b]-1,4-thiazin-4-yl)propanoic acid.HCl

t-Butyl 3-(2,3-dihydro-3-oxo-4H-pyrido[4,3-b]-1,4-thiazin-4-yl)propanoate (1.0g) was dissolved in DCM (dry, 5 ml) and treated with hydrogen chloride in dioxan (4M, 10ml). After 4h the mixture was evaporated in vacuo to give the title compound (0.75g).

NMR (δ) ($DMSOd_6$): 2.6 (2H, t), 3.75 (2H, s), 4.2 (2H, t), 7.95 (1H, dd), 8.4 (1H, dd), 8.85 (1H, s).

(c) Isopropyl 2-(1-(3-(2,3-dihydro-3-oxo-4H-pyrido[4,3-b]-1,4-thiazin-4-yl)propanoyl-Phe-Leu)amino-2-cyclohexylethyl)-1,3-dithiane-2-carboxylate

This material was prepared from 3-(2,3-dihydro-3-oxo-4H-pyrido[4,3-b]-1,4-thiazin-4-yl)propanoic acid.HCl (0.56g) following the procedure of Description 5(f). Chromatography on silica gel using ethyl acetate/petroleum ether (bp 60-80° C) (50-100% ethyl acetate, gradient) gave the title compound (1.2g) as a white foam.

NMR (δ) ($CD_3OD$): 0.7-0.85 (4H, m), 0.9-1.05 (4H, m), 1.1-1.45 (12H, m), 1.5-1.85 (8H, m), 1.85-2.15 (2H, m), 2.55 (2H, m), 2.6-2.75 (2H, m), 2.75-3.3 (4H, m), 3.5 (2H, m), 4.05-4.4 (2.5H, m), 4.45 (1H, m), 4.65 (2H, m), 4.95 (0.5H, s), 5.05 (1H, m), 7.1-7.3 (5H, m), 7.35 (0.5H, d), 7.45 (1H, m), 7.5-7.6 (0.5H, m), 8.05-8.2 (1.5H, m), 8.3 (0.5H, m), 8.4-8.5 (1H, m).

M.S. (m/z) (FAB) (M+1) = 812 (consistent with m.w.=811).

* Synth. Comm. 331-3 (1985).

Description 7

(a) Ethyl 4-(2,3-dihydro-3-oxo-4H-pyrido[4,3-b]-1,4-thiazin-4-yl) butanoate

This material was prepared from 2,3-dihydro-3-oxo-4H-pyrido[4,3-b]-1,4-thiazine (1.01g) and ethyl 4-bromobutyrate (1.0 ml) following the procedure of Description 5(d). Chromatography on silica gel using methanol/chloroform (0-5% methanol, gradient) gave the title compound (0.82g) as an orange solid.

NMR (δ) ($CDCl_3$): 1.25 (3H, t), 2.05 (2H, quin), 2.45 (2H, t), 3.45 (2H, s), 4.15 (4H, m), 7.3 (1H, m), 8.2 (1H, d), 8.55 (1H, s).

(b) 4-(2,3-Dihydro-3-oxo-4H-pyrido[4,3-b]-1,4-thiazin-4-yl)butanoic acid. HCl

This material was prepared from ethyl 4-(2,3-dihydro-3-oxo-4H-pyrido[4,3-b]-1,4-thiazin-4-yl)butanoate (0.79g) following the procedure of Description 5(e). This gave the title compound (0.66g) as a yellow solid.

NMR (δ) ($CDCl_3$): 2.0 (2H, m), 2.5 (2H, t), 3.65 (2H, s), 4.2 (2H, t), 7.8 (1H, d), 8.4 (1H, d), 8.8 (1H, s).

(c) Isopropyl 2-(1-(4-(2,3-dihydro-3-oxo-4H-pyrido-[4,3-b]-1,4-thiazin-4-yl)butanoyl-Phe-Leu)amino-2-cyclohexylethyl)-1,3-dithiane-2-carboxylate

This material was prepared from 4-(2,3-dihydro-3-oxo-4H-pyrido[4,3-b]-1,4-thiazin-4-yl)butanoic acid.HCl (0.27g) following the procedure of Description 5(f).

Description 8

(a) 1,3-Dithiane-2-carboxylic acid chloride

1,3-Dithiane-2-carboxylic acid (0.35g) (Description 1(a)) was stirred in toluene (dry, 10 ml) and DMF (2 drops). Oxalyl chloride (0.2 ml) was added slowly, causing effervescence and warming, and the mixture stirred for 1h. The mixture was evaporated in vacuo, diluted with diethyl ether, dried ($K_2CO_3$) and evaporated in vacuo to give the title compound (0.31g) as a yellow oil.

NMR (δ) ($CDCl_3$): 2.1 (2H, m), 2.65 (2H, m), 3.25 (2H, m), 4.4 (1H, s).

(b) 4-(1-Methylpiperidinyl) 1,3-dithiane-2-carboxylate

1,3-Dithiane-2-carboxylic acid chloride (0.31g) was stirred in DMF (dry, 10 ml) with 4-dimethylaminopyridine (0.21g) and 4-hydroxy-1-methylpiperidine (0.21g) for 19h. The mixture was diluted with ethyl acetate (50 ml), washed with water and brine, dried ($Na_2SO_4$) and evaporated in vacuo. The crude product was chromatographed on silica gel using methanol/chloroform (0-10% methanol, gradient) to give the title compound (0.20g) as an orange solid.

NMR (δ) ($CDCl_3$): 1.8 (2H, m), 1.9-2.4 (9H, m), 2.5-2.7 (4H, m), 3.4 (2H, m), 4.2 (1H, s), 4.9(1H, m).

(c) 4-(1-Methylpiperidinyl) 2-(1-(CBZ-amino)-2-cyclohexylethyl)-1,3-dithiane-2-carboxylate

This material was formed from 4-(1-methylpiperidin-yl)-1,3-dithiane-2-carboxylate (10.87g) and N-CBZ-2-cyclohexyl-1-(toluene-p-sulphonyl)ethylamine (Description 2(b)) (14.4g) following the procedure of Description 2(c). Chromatography on silica gel using methanol/chloroform (0-20% methanol, gradient) gave the title compound (6.11g) as an orange oil.

NMR (δ) ($DMSOd_6$): 0.7-1.3 (6H, m), 1.5-1.7 (7H, m), 1.75 (4H, m), 2.0 (2H, m), 2.15 (3H, m), 2.2 (2H, m), 2.7-2.95 (4H, m), 3.15 (2H, m), 4.3 (1H, t), 4.75 (1H, m), 4.95-5.2 (2H, m), 7.0 (1H, d), 7.35 (5H, m).

M.S. (m/z) (FAB) (M+1) = 521 (consistent with m.w. = 520).

(d) 4-(1-Methylpiperidinyl) 2-(1-(BOC-Phe-Leu)amino-2-cyclohexylethyl)-1,3-dithiane-2-carboxylate

This material was prepared from 4-(1-methylpiperidinyl)-2-(1-(CBZ-amino)-2-cyclohexylethyl)-1,3-dithiane -2-carboxylate (0.56g) and BOC-Phe-Leu-OH (0.45g), following the procedure of Description 3(d). Chromatography on silica gel using methanol/chloroform (0-5% methanol, gradient) gave the title compound (0.37g) as a yellow solid.

NMR (δ) ($CDCl_3$): 0.9 (8H, m), 1.0-1.3 (4H, m), 1.4 (9H, s), 1.5-2.2 (16H, m), 2.3-2.5 (5H, m), 2.7 (4H, m),

3.15 (4H, m), 4.25-4.5 (2H, m), 4.65 (1H, m), 4.9 (2H, m), 6.5 (2H, m), 7.2-7.4 (5H, m).

(e) 4-(1-Methylpiperidinyl)2-(1-(3-(2,3-dihydro-3-oxo-4H-pyrido[4,3-b]-1,4-thiazin-4-yl)propanoyl-Phe-Leu)-amino-2-cyclohexylethyl)-1,3-dithiane-2-carboxylate

This material was formed from 4-(1-methylpiperidinyl)-2-(1-(BOC-Phe-Leu)amino-2-cyclohexylethyl)-1,3-dithiane-2-carboxylate (0.37g) following the deprotection procedure of Description 6(b) and the coupling procedure of Description 3(d). Chromatography on silica gel using methanol/chloroform (0-10% methanol, gradient) gave the title compound (0.13g) as a white solid.

NMR (δ) ($DMSOd_6$): 0.6-0.95 (8H, m), 1.0-1.25 (5H, m), 1.3-1.7 (13H, m), 1.8 (3H, m), 2.0 (1H, m), 2.1-2.3 (5H, m), 2.45 (2H, m), 2.6-2.85 (3H, m), 2.85-3.15 (3H, m), 3.55 (2H, m), 3.9-4.05 (2H, m), 4.1-4.4 (1H, m), 4.55 (2H, m), 4.75 (1H, m), 7.1-7.3 (5H, m), 7.3-7.5 (2H, m), 8.15 (1H, m), 8.3 (2H, m), 8.5 (1H, m).

M.S. (m/z) (FAB) (M+1) = 867 (consistent with m.w. = 866).

Example 1

Isopropyl 3-((2,3-dihydro-1,1-dioxobenzothiophene-3-yl)acetyl-Phe-Leu-amino)-4-cyclohexyl-2-oxobutanoate

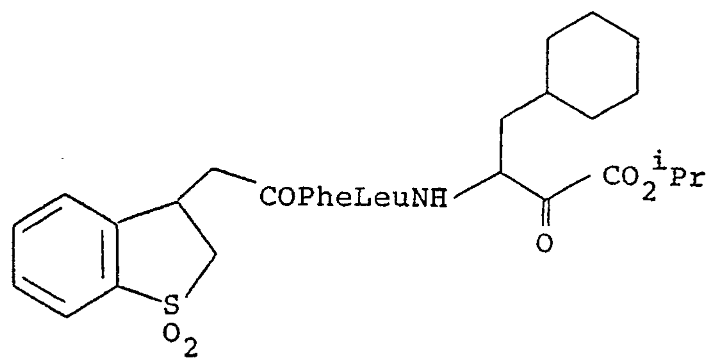

Isopropyl 2-(1-((2,3-dihydro-1,1-dioxobenzothiophene-3-yl)acetyl-Phe-Leu-amino)-2-cyclohexylethyl)-1,3-dithiane-2-carboxylate (Description 3) (0.34g) was stirred in acetonitrile (5 ml) as N-bromosuccinimide (NBS) (0.46g) was added in a mixture of acetonitrile (4 ml) and water (1 ml). The mixture was stirred for 5 min, diluted with ethyl acetate (50 ml), washed with dilute aqueous sodium metabisulphite (sufficient to decolourise), 5% aqueous sodium hydrogen carbonate, and brine, dried ($Na_2SO_4$) and evaporated in vacuo. Chromatography on silica gel using methanol/chloroform (0-2% methanol, gradient) gave the title compound (0.23g) as a white foam.

NMR (δ) ($DMSOd_6$): 0.6-1.0 (7H,m), 1.0-1.9 (21H,m), 2.45 (1H,m), 2.75 (2H,m), 2.9-3.15 (2H,m), 3.5 (1H,m), 3.8 (1H,m), 4.15-4.45 (2H,m), 4.65 (1H,m), 4.8 (1H,m), 5.05 (1H,m), 5.9-6.7 (several small m), 7.15-7.3 (5H,m), 7.35-7.65 (4H,m), 7.7 (1H,m), 8.0-8.5 (3H,m).

M.S. (m/z) (FAB) (M+1) = 710 (consistent with m.w. = 709).

Examples 2-6

The following compounds were prepared

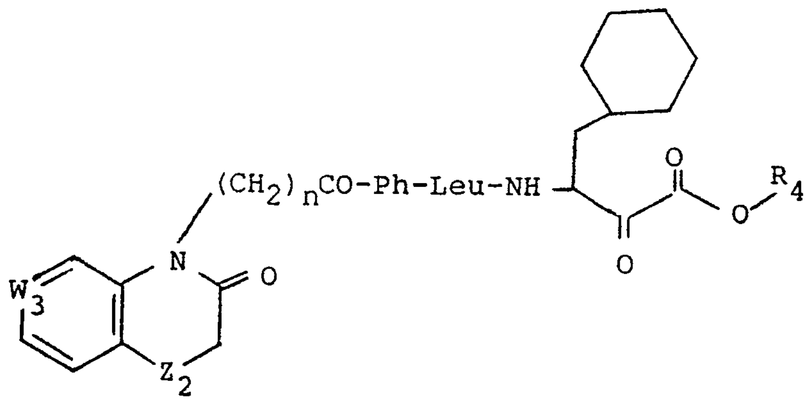

| Compound/ Ex. No. | $W_3$ | $Z_2$ | n | $R_4$ |
|---|---|---|---|---|
| Ex. 2 | BOC-$NHCH_2$-C | O | 2 | $^iPr$ |
| Ex. 3 | $H_2NCH_2$-C | O | 2 | $^iPr$ |
| Ex. 4 | N | S | 2 | $^iPr$ |
| Ex. 5 | N | S | 3 | $^iPr$ |
| Ex. 6 | N | S | 2 | * |

*4-(1-methylpiperidinyl)

Example 2

Isopropyl 3-(3-(6-(BOC-aminomethyl)-2,3-dihydro-3-oxo-4H-1,4-benzoxazin-4-yl)propanoyl-Phe-Leu)amino-4-cyclohexyl-2-oxobutanoate

This material was formed from isopropyl 2-(1-(3-(6-(BOC-aminomethyl)-2,3-dihydro-3-oxo-4H-1,4-benzoxazin-4-yl)propanoyl-Phe-Leu)amino-2-cyclohexylethyl)-1,3-dithiane-2-carboxylate (Description 5(f)) (0.21g) following the procedure of Example 1. Chromatography on silica gel using methanol/chloroform (0-5% methanol, gradient) gave the title compound (0.14g) as a white solid.

NMR (δ) ($CDCl_3$): 0.7-1.05 (8H, m), 1.05-1.4 (11H, m), 1.45 (9H, m), 1.5-1.8 (8H, m), 1.85 (1H, m), 2.55 (2H, m), 2.9-3.2 (2H, m), 4.0-4.65 (8H, m), 5.15 (2H, m), 6.25-6.7 (1H, m), 6.85-7.0 (2H, m), 7.0-7.35 (8H, m).

Example 3

Isopropyl 3-(3-(6-(aminomethyl)-2,3-dihydro-3-oxo-4H-1,4-benzoxazin-4-yl)propanoyl-Phe-Leu)amino-4-cyclohexyl-2-oxobutanoate.2TFA.1.5$H_2O$

This material was formed from isopropyl 3-(3-(6-(BOC-aminomethyl)-2,3-dihydro-3-oxo-4H-1,4-benzoxazin-4-yl)-propanoyl-Phe-Leu)amino-4-cyclohexyl-2-oxobutanoate(Example 2) (0.12g), following the procedure of Description 4(b). This gave the title compound (0.093g) as a white solid.

NMR (δ) ($DMSOd_6$): 0.7-1.0 (7H, m), 1.0-1.4 (12H, m), 1.4-1.9 (9H, m), 2.45 (m), 2.75 (1H, m), 2.85-3.1 (1H, m), 3.85-4.1 (3H, m), 4.1-4.4 (1H, m), 4.5-4.7 (3H, m), 4.7-4.85 (0.5H, m), 5.05 (0.5H, m), 6.0 (0.5H, m), 6.35 (0.5H, m), 7.0-7.1 (2H, m), 7.1-7.3 (5.5H, m), 7.35 (0.5H, m), 8.0-8.15 (2H, m), 8.15-8.5 (2H, m).

Analysis: $C_{40}H_{55}N_5O_8.C_2HF_3O_2.1.5H_2O$ requires C,57.7; H,6.8; N,8.00%. Found: C,57.6; H,6.7; N,7.7%.

M.S. (m/z) (FAB) (M+1) = 734 (consistent with m.w. of free base = 733).

Example 4

Isopropyl 3-(3-(2,3-dihydro-3-oxo-4H-pyrido[4,3-b]-1,4-thiazin-4-yl)propanoyl-Phe-Leu)amino-4-cyclohexyl-2-oxobutanoate.$2H_2O$

This material was formed from isopropyl 2-(1-(3-(2,3-dihydro-3-oxo-4H-pyrido[4,3-b]-1,4-thiazin-4-yl)-propanoyl-Phe-Leu)amino-2-cyclohexylethyl)-1,3-dithiane-2-carboxylate (Description 6(c)) (0.27g) following the procedure of Example 1. Chromatography on silica gel using methanol/chloroform (0-2% methanol, gradient) gave the title compound (0.12g) as a white solid.

NMR (δ) ($DMSOd_6$): 0.65-1.0 (8H, m), 1.0-1.3 (10H, m), 1.35 (2H, m), 1.4-1.95 (8H, m), 2.45 (2H, m), 2.75 (1H, m), 2.9 (1H, m), 3.1 (0.5N, m), 3.55 (2H, dd), 3.9-4.1 (2H, m), 4.25 (1H, m), 4.35 (1H, m), 4.55 (1H, m), 4.75 (1H, m), 5.0 (0.5H, m), 7.1-7.3 (5H, m), 7.45 (1H, m), 8.05 (0.5H, m), 8.15 (1H, dd), 8.2-8.35 (1.5H, m), 8.35-8.45 (0.5H, m), 8.5 (1H, m).

Analysis: $C_{38}H_{51}N_5O_9S.2H_2O$ requires C,60.2; H,7.3; N,9.2%. Found: C,60.1; H,7.0; N,9.1%.

M.S. (m/z) (FAB) (M+1) = 722 (consistent with m.w. = 721).

Example 5

Isopropyl 3-(4-(2,3-dihydro-3-oxo-4H-pyrido[4,3-b]-1,4-thiazin-4-yl)butanoyl-Phe-Leu)amino-4-cyclohexyl-2-oxobutanoate

This material was formed from isopropyl 2-(1-(4-(2,3-dihydro-3-oxo-4H-pyrido[4,3-b]-1,4-thiazin-4-yl)-butanoyl-Phe-Leu)amino-2-cyclohexylethyl)-1,3-dithiane-2-carboxylate (Description 7(c)) (0.22g), following the procedure of Example 4.

Analysis: $C_{39}H_{53}N_5O_7S.2H_2O$ requires C,60.7; H,7.4; N,9.1% Found: C,60.7; H,7.5; N,9.1%

M.S. (m/z)(FAB)(M+1)= 736 (consistent with m.w. of free base = 735).

Example 6

4-(1-Methylpiperidinyl) 3-(3-(2,3-dihydro-3-oxo-4H-pyrido[4,3-b]-1,4-thiazin-4-yl)propanoyl-Phe-Leu)amino-4-cyclohexyl-2-oxobutanoate

This material was formed from 4-(1-methylpiperidinyl)2-(1-(3-(2,3-dihydro-3-oxo-4H-pyrido[4,3-b]-1,4-thiazin-4-yl)propanyl-Phe-Leu)amino-2-cyclohexylethyl)-1,3-dithiane-2-carboxylate (Description 8(e)) (0.37g), following the procedure of Example 4.

M.S. (m/z) (FAB) (M+1) = 777 (consistent with m.w. of free base = 776).

Biological Data

In vitro human renin inhibition

Renin inhibitory activity was estimated as the percentage change in renin activity in human plasma in the presence and absence of compound. The source of plasma was blood taken from healthy volunteers. Renin activity was defined by the difference in angiotensin I levels between two halves of a sample, one incubated at 37° and the other at 4° for 2 h. Angiotensin I levels were measured using a $^{125}I$- angiotensin I radioimmuno- assay kit (NEN/DuPont, Stevenage). Results were calculated as the mean of at least two, duplicate determinations and $IC_{50}$ values were calculated by linear regression analysis of at least three concentrations of compound.

The results were as follows:

| Compound | $IC_{50}$ (nm) |
|---|---|
| E1 | 15 |
| E3 | 34 |
| E4 | 5 |

## Claims

1. A compound of formula (I), or a pharmaceutically acceptable salt thereof:

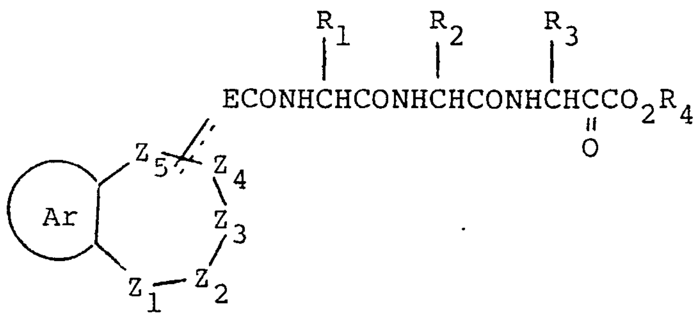

(I)

wherein
the Ar ring is of sub-formula (a) or (b):

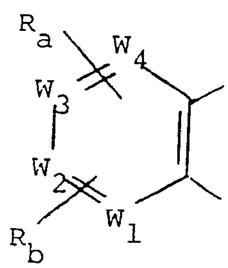

(a)

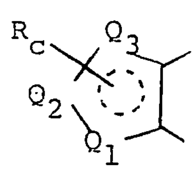

(b)

one or two of $W_1$, $W_2$, $W_3$ and $W_4$ is CH, N or NO (such that if two are NO then these are are $W_1$ and $W_4$), and the others are CH, $CR_a$ or $CR_b$;
one of $Q_1$, $Q_2$ and $Q_3$ is S and the other two are CH and $CR_c$;
either $Z_1$ and $Z_2$ are absent and $Z_3$, $Z_4$ and $Z_5$ and the carbon atoms to which $Z_3$ and $Z_5$ are attached, form a 5-membered non-aromatic heterocyclic ring; or
$Z_1$ is absent and $Z_2$, $Z_3$, $Z_4$, $Z_5$ and the carbon atoms to which $Z_2$ and $Z_5$ are attached, form a 6-membered non-aromatic heterocyclic ring; or

$Z_1$, $Z_2$, $Z_3$, $Z_4$ and $Z_5$ and the carbon atoms to which $Z_1$ and $Z_5$ are attached, form a 7-membered non-aromatic heterocyclic ring;
E is absent or is $(CH_2)_n$ or $CH(CH_2)_{n-1}$ wherein n is 1 to 4;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl or $C_{3-8}$ cycloalkyl or phenyl;
$R_4$ is $C_{1-6}$ alkyl, $C_{3-8}$ cycloalkyl, $C_{3-8}$ cycloalkyl-$C_{1-4}$ alkyl or a group of structure c), d), e) or f):

c) 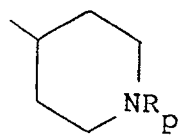

d) 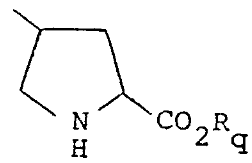

e) 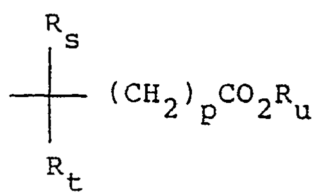

f) 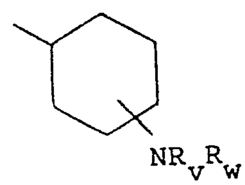

wherein
$R_p$, $R_q$, $R_s$, $R_t$, $R_u$, $R_v$ and $R_w$ are selected from hydrogen or $C_{1-6}$ alkyl; or $NR_vR_w$ is 1-imidazolyl; and the dashed line represents an optional bond (when E is present).

**2.** A compound according to claim 1 wherein $Z_1$ and $Z_2$ are absent, $Z_3$ is CO, $Z_4$ is N, $Z_5$ is CH and E is attached at $Z_4$.

**3.** A compound according to claim 1 wherein the Ar ring is of sub-formula (a), $W_3$ is N and $W_1$, $W_2$ and $W_4$ are CH, $CR_a$ or $CR_b$, $Z_2$ is S, $Z_3$ is $CH_2$, $Z_4$ is CO, $Z_5$ is N and E is attached at $Z_5$.

**4.** A compound according to any one of claims 1 to 3 wherein one of $R_a$ and $R_b$ in sub-formula (b) is hydrogen and the other is $CH_2NH_2$, $CO_2H$ or $CH_2NHCH_2CO_2H$, or $R_c$ in sub-formula (b) is hydrogen.

**5.** A compound according to any one of claims 1 to 8 wherein the amino acid residue containing $R_2$ is Leu, $\beta$-pyrazolylalanine or His.

**6.** A compound according to claim 1 of formula (IA) or a pharmaceutically acceptable salt thereof:

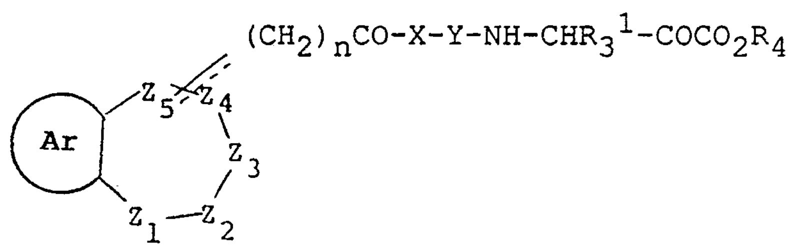

(IA)

wherein X is Phe;
Y is Leu or His;
$R_3{}^1$ is cyclohexylmethyl; and
the remaining variables are as defined in claim 1.

**7.** A compound according to any one of claims 1 to 10 wherein E is $(CH_2)_n$ and E is attached at $Z_4$ or $Z_5$.

**8.** A compound according to any one of claims 1 to 11 wherein the carbon atom in $R_4$ adjacent $CO_2$, is either a secondary or tertiary carbon atom.

**9.** A compound according to claim 1, selected from the group consisting of

isopropyl 3-((2,3-dihydro-1,1-dioxobenzothiophene-3-yl)acetyl-Phe-Leu-amino)-4-cyclohexyl-2-oxobutanoate,

isopropyl 3-(3-(6-(BOC-aminomethyl)-2,3-dihydro-3-oxo-4H-1,4-benzoxazin-4-yl)propanoyl-Phe-Leu)-amino-4-cyclohexyl-2-oxobutanoate,

isopropyl 3-(3-(6-(aminomethyl)-2,3-dihydro-3-oxo-4H-1,4-benzoxazin-4-yl)propanoyl-Phe-Leu)amino-4-cyclohexyl-2-oxobutanoate,

isopropyl 3-(3-(2,3-dihydro-3-oxo-4H-pyrido[4,3-b]-1,4-thiazin-4-yl)propanoyl-Phe-Leu)amino-4-cyclohexyl-2-oxo-butanoate,

isopropyl 3-(4-(2,3-dihydro-3-oxo-4H-pyrido[4,3-b]-1,4-thiazin-4-yl)butanoyl-Phe-Leu)amino-4-cyclohexyl-2-oxo-butanoate, and

4-(1-methylpiperidinyl) 3-(3-(2,3-dihydro-3-oxo-4H-pyrido[4,3-b]-1,4-thiazin-4-yl)propanoyl-Phe-Leu)-amino-4-cyclohexyl-2-oxobutanoate.

**10.** A process for the preparation of a compound according to claim 1, which process comprises reacting a reagent of formula (III):

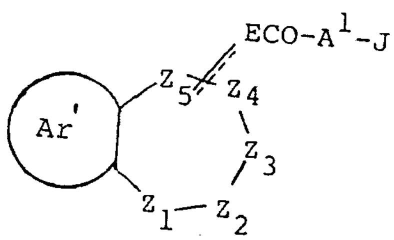

(III)

wherein Ar' is Ar as defined in claim 1, but substituted by $R_a'$, $R_b'$ in sub-formula (a) and by $R_c'$ in sub-formula (b), wherein $R_a'$, $R_b'$ and $R_c'$ are $R_a$, $R_b$ and $R_c$ respectively or groups or atoms convertible thereto. $A^1$ is absent or represents an appropriate amino acid or dipeptide unit; J is OH or a leaving group; and the remaining variables are as hereinbefore defined; with a reagent of the formula (IV):

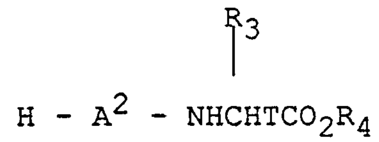

(IV)

wherein

$A^2$ is absent or represents an appropriate amino acid or dipeptide unit, such that $A^1$ + $A^2$ is $-NHCHR_1CONHCHR_2CO-$; T is an optionally protected carbonyl group; and the remaining variables are as hereinbefore defined; and thereafter, if desired or necessary deprotecting (T or within $A^1$ or $A^2$) of the products, and/or converting $Z_1$, $Z_2$, $Z_3$, $Z_4$ or $Z_5$ to other $Z_1$, $Z_2$, $Z_3$, $Z_4$ or $Z_5$, $R_a'/R_b'/R_c'$ in Ar' to $R_a$, $R_b$, and/or $R_c$ and/or $W_1$, $W_2$, $W_3$ or $W_4$ in Ar' when N to NO; and/or forming a pharmaceutically acceptable salt thereof.

**11.** A pharmaceutical composition comprising a compound according to any one of claims 1 to 9, and a pharmaceutically acceptable carrier.

**12.** A compound according to any one of claims 1 to 9 for use as an active therapeutic substance.

**13.** A compound according to any one of claims 1 to 9 for use in the treatment of hypertension.

**14.** Use of a compound according to any one of claims 1 to 9 in the manufacture of a medicament for use in the treatment of hypertension.

**Claims for the following Contracting State: ES**

**1.** A process for the preparation of a compound of formula (I), or a pharmaceutically acceptable salt thereof:

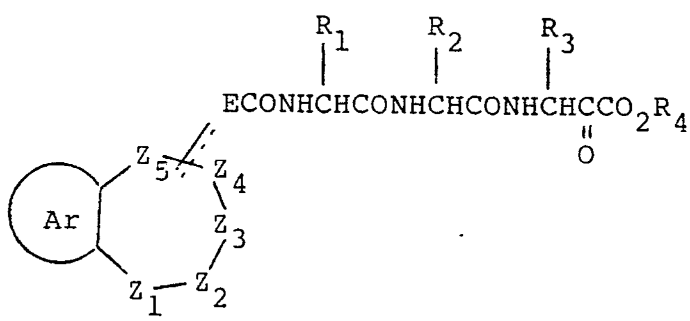

(I)

wherein

the Ar ring is of sub-formula (a) or (b):

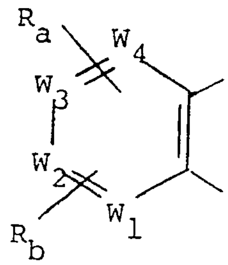

(a)

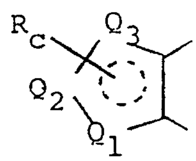

(b)

one or two of $W_1$, $W_2$, $W_3$ and $W_4$ is CH, N or NO (such that if two are NO then these are are $W_1$ and $W_4$), and the others are CH, $CR_a$ or $CR_b$;
one of $Q_1$, $Q_2$ and $Q_3$ is S and the other two are CH and $CR_c$;
either $Z_1$ and $Z_2$ are absent and $Z_3$, $Z_4$ and $Z_5$ and the carbon atoms to which $Z_3$ and $Z_5$ are attached, form a 5-membered non-aromatic heterocyclic ring; or
$Z_1$ is absent and $Z_2$, $Z_3$, $Z_4$, $Z_5$ and the carbon atoms to which $Z_2$ and $Z_5$ are attached, form a 6-membered non-aromatic heterocyclic ring; or
$Z_1$, $Z_2$, $Z_3$, $Z_4$ and $Z_5$ and the carbon atoms to which $Z_1$ and $Z_5$ are attached, form a 7-membered non-aromatic heterocyclic ring;
E is absent or is $(CH_2)_n$ or $CH(CH_2)_{n-1}$ wherein n is 1 to 4;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl or $C_{3-8}$ cycloalkyl or phenyl;
$R_4$ is $C_{1-6}$ alkyl, $C_{3-8}$ cycloalkyl, $C_{3-8}$ cycloalkyl-$C_{1-4}$ alkyl or a group of structure c), d), e) or f):

c) 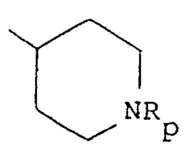

d) 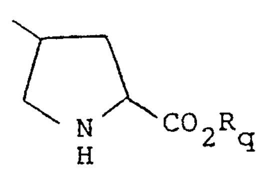

e) 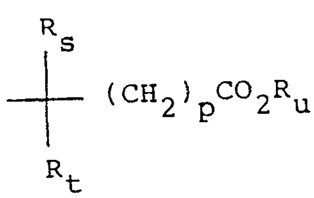

f) 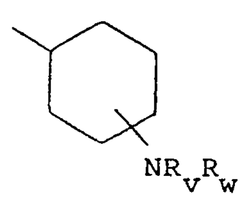

wherein
$R_p$, $R_q$, $R_s$, $R_t$, $R_u$, $R_v$ and $R_w$ are selected from hydrogen or $C_{1-6}$ alkyl; or $NR_vR_w$ is 1-imidazolyl; and

the dashed line represents an optional bond (when E is present); which process comprises reacting a reagent of formula (III):

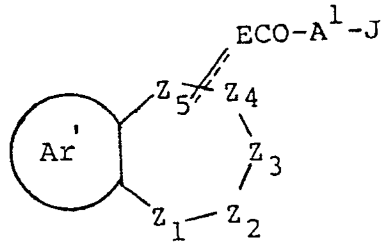

(III)

wherein Ar' is Ar as defined in claim 1, but substituted by $R_a'$, $R_b'$ in sub-formula (a) and by $R_c'$ in sub-formula (b), wherein $R_a'$, $R_b'$ and $R_c'$ are $R_a$, $R_b$ and $R_c$ respectively or groups or atoms convertible thereto. $A^1$ is absent or represents an appropriate amino acid or dipeptide unit; J is OH or a leaving group; and the remaining variables are as hereinbefore defined; with a reagent of the formula (IV):

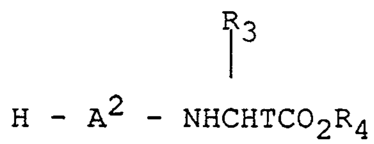

(IV)

wherein

$A^2$ is absent or represents an appropriate amino acid or dipeptide unit, such that $A^1$ + $A^2$ is $-NHCHR_1CONHCHR_2CO-$; T is an optionally protected carbonyl group; and the remaining variables are as hereinbefore defined; and thereafter, if desired or necessary deprotecting (T or within $A^1$ or $A^2$ ) of the products, and/or converting $Z_1$, $Z_2$, $Z_3$, $Z_4$ or $Z_5$ to other $Z_1$, $Z_2$, $Z_3$, $Z_4$ or $Z_5$, $R_a'/R_b'/R_c'$ in Ar' to $R_a$, $R_b$, and/or $R_c$ and/or $W_1$, $W_2$, $W_3$ or $W_4$ in Ar' when N to NO; and/or forming a pharmaceutically acceptable salt thereof.

**2.** A process according to claim 1 wherein $Z_1$ and $Z_2$ are absent, $Z_3$ is CO, $Z_4$ is N, $Z_5$ is CH and E is attached at $Z_4$.

**3.** A process according to claim 1 wherein the Ar ring is of sub-formula (a), $W_3$ is N and $W_1$, $W_2$ and $W_4$ are CH, $CR_a$ or $CR_b$, $Z_2$ is S, $Z_3$ is $CH_2$, $Z_4$ is CO, $Z_5$ is N and E is attached at $Z_5$.

**4.** A process according to any one of claims 1 to 3 wherein one of $R_a$ and $R_b$ in sub-formula (b) is hydrogen and the other is $CH_2NH_2$, $CO_2H$ or $CH_2NHCH_2CO_2H$, or $R_c$ in sub-formula (b) is hydrogen.

**5.** A process according to any one of claims 1 to 8 wherein the amino acid residue containing $R_2$ is Leu, $\beta$-pyrazolylalanine or His.

**6.** A process according to claim 1 for the preparation of a compound of formula (IA) or a pharmaceutically acceptable salt thereof:

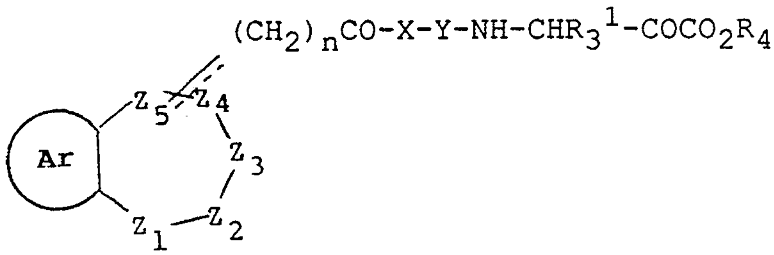

(IA)

wherein
X is Phe;
Y is Leu or His;
$R_3^1$ is cyclohexylmethyl; and
the remaining variables are as defined in claim 1.

7. A process according to any one of claims 1 to 10 wherein E is $(CH_2)_n$ and E is attached at $Z_4$ or $Z_5$.

8. A process according to any one of claims 1 to 11 wherein the carbon atom in $R_4$ adjacent $CO_2$, is either a secondary or tertiary carbon atom.

9. A process according to claim 1, for the preparation of a compound selected from the group consisting of

isopropyl 3-((2,3-dihydro-1,1-dioxobenzothiophene-3-yl)acetyl-Phe-Leu-amino)-4-cyclohexyl-2-oxobutanoate,

isopropyl 3-(3-(6-(BOC-aminomethyl)-2,3-dihydro-3-oxo-4H-1,4-benzoxazin-4-yl)propanoyl-Phe-Leu)-amino-4-cyclohexyl-2-oxobutanoate,

isopropyl 3-(3-(6-(aminomethyl)-2,3-dihydro-3-oxo-4H-1,4-benzoxazin-4-yl)propanoyl-Phe-Leu)amino-4-cyclohexyl-2-oxobutanoate,

isopropyl 3-(3-(2,3-dihydro-3-oxo-4H-pyrido[4,3-b]-1,4-thiazin-4-yl)propanoyl-Phe-Leu)amino-4-cyclohexyl-2-oxo-butanoate,

isopropyl 3-(4-(2,3-dihydro-3-oxo-4H-pyrido[4,3-b]-1,4-thiazin-4-yl)butanoyl-Phe-Leu)amino-4-cyclohexyl-2-oxo-butanoate, and

4-(1-methylpiperidinyl) 3-(3-(2,3-dihydro-3-oxo-4H-pyrido[4,3-b]-1,4-thiazin-4-yl)propanoyl-Phe-Leu)-amino-4-cyclohexyl-2-oxobutanoate.

10. Use of a compound of formula (I) as defined in claim 1, or a pharmaceutically acceptable salt thereof, in the manufacture of a medicament for use in the treatment of hypertension.

European Patent Office

# EUROPEAN SEARCH REPORT

Application Number

**EP 90 31 3104**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 296 581 (SQUIBB & SONS INC.)<br>* Claims 1,10,11 *<br>- - - - - | 1-10 | C 07 K 5/02<br>C 07 K 5/06<br>A 61 K 37/64 |

TECHNICAL FIELDS SEARCHED (Int. Cl.5)

C 07 K
A 61 K

The present search report has been drawn up for all claims

| Place of search | Date of completion of search | Examiner |
|---|---|---|
| The Hague | 14 March 91 | DEFFNER C-A.E. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document